# EUROPEAN PATENT APPLICATION

(11) **EP 4 759 823 A1**
(43) Date of publication of application: **17.06.2026**
(21) Application number: 24865890.8
(22) Date of filing: 12.09.2024
(51) Int. Cl.: C07K 5/113, A61P 17/14, A61K 8/64, A61Q 7/00, A61K 38/00

(54) **PEPTIDE HAVING ACTIVITIES OF PROMOTING HAIR GROWTH AND INHIBITING HAIR LOSS, AND USES THEREOF**

(30) Priority: 15.09.2023 KR 20230122858
(71) Applicant: Caregen Co., Ltd., Seoul 06188 (KR)
(72) Inventor: CHUNG, Yong Ji, Seoul 06188 (KR); KIM, Eun Mi, Seoul 06188 (KR); LEE, Eung Ji, Seoul 06188 (KR); KIM, Min Woong, Seoul 06188 (KR); GIL, Hana, Seoul 06188 (KR)
(74) Representative: Bandpay & Greuter
(86) International application number: PCT/KR2024/013948
(87) International publication number: WO 2025/058451

(57) **Abstract**

The present invention relates to a peptide having the activities of promoting hair growth, and preventing and inhibiting alopecia, and uses thereof. The peptide of the present invention has the activities of: promoting the proliferation of hair follicle dermal papilla cells (HFDPC), activating cell proliferation-related signaling proteins and beta-catenin in human dermal papilla cells, enhancing the expression levels of growth factor-related genes, enhancing the expression levels of beta-catenin downstream genes, downregulating the expression level of hair loss protein DKK-1, increasing the expression of keratin proteins in hair outer root sheath cells (HORSC), and increasing the expression of transcription factors involved in cell activation in germinal matrix cells (GMC); and promoting the differentiation of hair follicle stem cells (HFSC).

## Description

### TECHNICAL FIELD

### [CROSS-REFERENCE TO RELATED APPLICATIONS]

This application claims priority from Korean Patent Application No. 10-2023-0122858, filed on September 15, 2023, the disclosure of which is incorporated herein by reference in its entirety.

### [TECHNICAL FIELD]

The present invention relates to a peptide having activities of promoting hair growth and inhibiting hair loss, and uses thereof.

### BACKGROUND ART

Alopecia refers to a phenomenon in which hair shedding occurs excessively due to a prolongation of the telogen phase beyond a normal level in the hair growth cycle or an increase in the number of hair follicles in the telogen phase, and in which hair is excessively shed and is not restored due to stress, nutritional deficiency, local blood flow disorder, hormonal and genetic factors, or the like.

In addition to endogenous alopecia such as androgenic alopecia and seborrheic alopecia, exogenous alopecia caused by factors such as ultraviolet rays or fine particulate matter is known. Patients with alopecia strongly complain of psychological stress caused by changes in external appearance, rather than of functional losses of hair such as protection against external stimuli.

As therapeutic agents for alopecia, 5α-reductase inhibitors that act to convert testosterone, which is a male hormone, into dihydrotestosterone (DHT), which is an androgen, for example, drugs such as finasteride or dutasteride, have been developed and approved for marketing, and minoxidil, which is a drug having an action of promoting hair growth by stimulating hair follicles and increasing blood flow, has also been approved and is in use. However, in the case of 5α-reductase inhibitors, side effects such as sexual dysfunction, depression, anxiety, and gynecomastia have been reported to occur relatively frequently, and in the case of minoxidil as well, cardiovascular side effects such as electrocardiographic abnormalities, tachycardia, and endocarditis have been reported to occur; moreover, minoxidil is known to cause the recurrence of hair loss when administration of the drug is discontinued.

Peptides have high biocompatibility, and when used as a single active ingredient in a drug or cosmetic composition, have fewer side effects than extracts or the like containing various ingredients. In addition, after locally exerting an effect of inhibiting hair loss, peptides can be easily degraded and eliminated by proteolytic enzymes in the body; therefore, unlike conventional therapeutic agents, peptides are less likely to raise concerns regarding side effects.

International Patent Application WO 2005-082395 discloses a peptide having an effect of promoting hair growth by promoting progression from a telogen phase to a growth phase in a hair growth cycle, and Korean Patent No. 10-2387764 discloses a dimeric peptide derivative having an action of promoting proliferation of hair follicle cells.

### [Related Art Documents]

### [Patent Documents]

WO 2005-082395
Korean Patent No. 10-2387764

### DISCLOSURE OF THE INVENTION

### TECHNICAL PROBLEM

The present inventors have made research efforts to develop a peptide having improved activity for promoting hair growth and inhibiting hair loss, which is effective in promoting hair growth and has reduced side effects. As a result, the present inventors confirmed that a novel peptide developed by the present inventors promotes proliferation of human hair follicle dermal papilla cells (HHFDPCs), activates cell proliferation-related signaling proteins in human hair follicle dermal papilla cells, upregulates gene expression of growth factors, activates β-catenin, upregulates expression levels of β-catenin downstream genes, and downregulates an expression level of DKK-1, which is a hair loss-inducing protein. In addition, it was experimentally demonstrated that the peptide of the present invention increases expression of keratin proteins in hair outer root sheath cells (HORSCs), increases expression of MSX2, HOXC13, and FOXN1, which are transcription factors involved in cellular activity, in human hair germinal matrix cells (HHGMCs), and promotes proliferation of hair follicle stem cells (HFSCs) and induces differentiation thereof, thereby completing the present invention.

An object of the present invention is to provide a novel peptide having activities of promoting hair growth and inhibiting hair loss.

Another object of the present invention is to provide a composition for promoting hair growth or inhibiting, preventing, or alleviating alopecia, the composition containing the peptide as an active ingredient.

Still another of the present invention is to provide a pharmaceutical composition for preventing or treating alopecia, the pharmaceutical composition containing the peptide as an active ingredient.

Still another object of the present invention is to provide a cosmetic composition for promoting hair growth or preventing or alleviating alopecia, the cosmetic composition containing the peptide as an active ingredient.

Still another object of the present invention is to provide a method for promoting hair growth or inhibiting, preventing, alleviating, or treating alopecia, the method including applying the peptide or a composition containing the peptide as an active ingredient to a subject.

### TECHNICAL SOLUTION

One aspect of the present invention provides a peptide comprising the amino acid sequence of SEQ ID NO: 1.

Another aspect of the present invention provides a composition for promoting hair growth or inhibiting, preventing, or alleviating alopecia, the composition containing a peptide comprising the amino acid sequence of SEQ ID NO: 1 as an active ingredient.

Still another aspect of the present invention provides a pharmaceutical composition for preventing or treating alopecia, the pharmaceutical composition containing a peptide comprising the amino acid sequence of SEQ ID NO: 1 as an active ingredient.

Still another aspect of the present invention provides a cosmetic composition for promoting hair growth or preventing or alleviating alopecia, the cosmetic composition containing a peptide comprising the amino acid sequence of SEQ ID NO: 1 as an active ingredient.

Still another object of the present invention is to provide a method for promoting hair growth or inhibiting, preventing, alleviating, or treating alopecia, the method including applying a peptide comprising the amino acid sequence of SEQ ID NO: 1 or a composition containing the peptide as an active ingredient to a subject.

Hereinafter, the present invention will be described in detail.

### 1. Peptide and Activity Thereof

According to one aspect of the present invention, the present invention provides a peptide comprising the amino acid sequence set forth in SEQ ID NO: 1.

As used herein, the term "peptide" refers to a linear molecule formed by amino acid residues linked to each other by peptide bonds.

The peptide comprising the amino acid sequence of SEQ ID NO: 1 of the present invention may be used without modification; however, amino acid variants or fragments having different sequences may also be used, provided that such variants or fragments are formed by deletion, insertion, substitution, or a combination thereof of amino acid residues within a range that does not affect the inherent activity of the peptide, such as activity of promoting hair growth or inhibiting alopecia.

The peptide of the present invention may be modified by phosphorylation, sulfation, acrylation, glycosylation, methylation, farnesylation, or the like, within a range that does not alter the activity thereof.

The peptide of the present invention includes a peptide comprising the amino acid sequence of SEQ ID NO: 1, a peptide comprising an amino acid sequence substantially identical thereto, and variants thereof or active fragments thereof. The "substantially identical amino acid sequence" refers to an amino acid sequence having 75% or more, for example, 80% or more, 85% or more, 90% or more, 95% or more, 97% or more, or 99% or more sequence identity to the amino acid sequence of SEQ ID NO: 1. In addition, the peptide may further include a targeting sequence, a tag, a labeled residue, or an amino acid sequence prepared for a specific purpose of increasing a half-life or peptide stability.

In one embodiment, the peptide of the present invention may be a peptide consisting of the amino acid sequence of SEQ ID NO: 1.

The peptide of the present invention may be a peptide in which N-terminal and/or C-terminal modification is induced by selecting a partial region of an amino acid sequence in order to increase the activity thereof. Through such N-terminal and/or C-terminal modification, stability of the peptide of the present invention may be significantly improved, and, for example, a half-life of the peptide may be increased upon administration in vivo. The term "stability" includes not only in vivo stability for protecting the peptide of the present invention from attack by proteases in the body, but also storage stability (for example, stability during storage at room temperature).

The N-terminal modification may be one in which a protecting group selected from the group consisting of an acetyl group, a fluorenylmethoxycarbonyl group, a formyl group, a palmitoyl group, a myristyl group, a stearyl group, and polyethylene glycol (PEG) is attached to the N-terminus of the peptide. The C-terminal modification may be one in which a hydroxyl group (-OH), an amino group (-NH₂), an azide (-NHNH2), or the like is attached to the C-terminus of the peptide, but is not limited thereto.

The peptide of the present invention may be prepared by various methods well known in the technical field to which the present invention pertains. For example, the peptide of the present invention may be prepared according to chemical synthesis methods known in the art, in particular, solid-phase synthesis techniques (Merrifield, J. Amer. Chem. Soc. 85:2149-54 (1963); Stewart et al., Solid Phase Peptide Synthesis, 2nd ed., Pierce Chem. Co., Rockford, 111 (1984)) or liquid-phase synthesis techniques (U.S. Patent No. 5,516,891).

The peptide of the present invention has activity of promoting hair growth or preventing or inhibiting alopecia.

In one embodiment, the peptide of the present invention promotes proliferation of hair follicle dermal papilla cells (HFDPCs).

In one embodiment, the peptide of the present invention promotes proliferation of hair outer root sheath cells (HORSCs).

In one embodiment, the peptide of the present invention promotes proliferation of hair follicle stem cells (HFSCs).

In one embodiment, the peptide of the present invention activates Akt or ERK, which are proteins involved in signaling, in hair follicle dermal papilla cells (HFDPCs).

In one embodiment, the peptide of the present invention upregulates expression of growth factors in hair follicle dermal papilla cells (HFDPCs). The growth factors may be one or more selected from the group consisting of basic fibroblast growth factor (bFGF), hepatocyte growth factor (HGF), keratinocyte growth factor (KGF), and vascular endothelial growth factor (VEGF).

In one embodiment, the peptide of the present invention activates β-catenin in hair follicle dermal papilla cells (HFDPCs) to promote nuclear translocation thereof.

In one embodiment, the peptide of the present invention upregulates gene expression of one or more factors selected from the group consisting of LEF-1, c-Myc, and Cyclin D1, which are downstream factors of β-catenin, in hair follicle dermal papilla cells (HFDPCs).

In one embodiment, the peptide of the present invention downregulates an expression level of DKK-1 (Dickkopf-1), which is a hair loss-inducing protein, in hair follicle dermal papilla cells (HFDPCs). It is known that, in hair follicles, dihydrotestosterone (DHT) promotes expression of DKK-1 (Dickkopf-1), and DKK-1 induced and produced by DHT inhibits proliferation of keratinocytes in hair follicles and induces apoptosis thereof, thereby promoting hair loss (J of Invest Dermatolo, 2008, Feb., 128(2), pp. 262-269). The hair loss-inducing protein DKK-1 is a protein whose expression level is increased by dihydrotestosterone (DHT), and the peptide of the present invention may downregulate the expression level of DKK-1 that is increased by DHT.

In one embodiment, the peptide of the present invention increases gene expression of one or more proteins selected from the group consisting of Ha3-II, Keratin 5, Keratin 14, and Keratin 19 in human hair outer root sheath cells (HHORSCs).

In one embodiment, the peptide of the present invention increases expression of one or more transcription factors selected from the group consisting of MSX2, HOXC13, and FOXN1 in human hair germinal matrix cells (HHGMCs).

In one embodiment, the peptide of the present invention downregulates expression of one or more stem cell markers selected from the group consisting of CD34, CD133, Keratin 15, and LHX2 in hair follicle stem cells (HFSCs).

In one embodiment, the peptide of the present invention upregulates expression of one or more stem cell differentiation markers selected from the group consisting of α-SMA, Keratin 75, Vimentin, Versican, and Nexin-1 in hair follicle stem cells (HFSCs).

Through the above-described activities, the peptide of the present invention may exhibit efficacy in promoting hair growth or inhibiting, preventing, or alleviating alopecia.

### 2. Composition for Promoting Hair Growth or Preventing, Alleviating, or Treating Alopecia

In another aspect of the present invention, there is provided a composition for promoting hair growth or preventing, alleviating, or treating alopecia, the composition containing a peptide comprising the amino acid sequence of SEQ ID NO: 1 as an active ingredient.

In still another aspect of the present invention, there is provided a composition for use in promoting hair growth or preventing, alleviating, or treating alopecia, the composition containing a peptide comprising the amino acid sequence of SEQ ID NO: 1 as an active ingredient.

As described above, the peptide comprising the amino acid sequence of SEQ ID NO: 1 of the present invention has activity of promoting hair growth or inhibiting or preventing alopecia.

In still another aspect of the present invention, there is provided a pharmaceutical composition for preventing or treating alopecia, the pharmaceutical composition containing a peptide comprising the amino acid sequence of SEQ ID NO: 1 as an active ingredient.

In still another aspect of the present invention, there is provided a pharmaceutical composition for use in preventing or treating alopecia, the pharmaceutical composition containing a peptide comprising the amino acid sequence of SEQ ID NO: 1 as an active ingredient.

The peptide comprising the amino acid sequence of SEQ ID NO: 1 is as described in the section "1. Peptide and Activity Thereof".

As used herein, the term "alopecia" refers to a condition or disease in which hair is absent from an area where hair should normally be present, or in which the number of hairs is reduced compared to a normal state, due to abnormal hair loss. In the present invention, the term "alopecia" may be used interchangeably with the term "hair loss". In one embodiment, hair loss or symptoms of hair loss in the present invention include hair loss on the scalp.

In the present invention, hair loss includes, but is not limited to, hereditary androgenetic alopecia (male pattern hair loss), female pattern hair loss, telogen effluvium, anagen effluvium, alopecia areata, tinea capitis caused by fungal infection, hair loss caused by hypothyroidism or hyperthyroidism, hair loss caused by inflammation (seborrheic dermatitis), hair loss caused by malnutrition, hair loss caused by diabetes, hair loss caused by lupus, hair loss caused by disorders of hair formation, hair loss caused by drug administration (anticoagulants, antidepressants, discontinuation of oral contraceptives, or chemotherapy), folliculitis decalvans, lichen planopilaris, hair loss caused by burns or trauma, hair loss caused by ultraviolet radiation, or hair loss caused by contact with airborne fine particulate matter.

In one embodiment, a composition or a pharmaceutical composition containing the peptide of the present invention as an active ingredient promotes proliferation of hair follicle dermal papilla cells (HFDPCs).

In one embodiment, a composition or a pharmaceutical composition containing the peptide of the present invention as an active ingredient promotes proliferation of hair outer root sheath cells (HORSCs).

In one embodiment, a composition or a pharmaceutical composition containing the peptide of the present invention as an active ingredient promotes proliferation of hair follicle stem cells (HFSCs).

In one embodiment, a composition or a pharmaceutical composition containing the peptide of the present invention as an active ingredient activates Akt or ERK, which are proteins involved in signaling, in hair follicle dermal papilla cells (HFDPCs).

In one embodiment, a composition or a pharmaceutical composition containing the peptide of the present invention as an active ingredient upregulates expression of growth factors in hair follicle dermal papilla cells (HFDPCs). The growth factors may be one or more selected from the group consisting of basic fibroblast growth factor (bFGF), hepatocyte growth factor (HGF), keratinocyte growth factor (KGF), and vascular endothelial growth factor (VEGF).

In one embodiment, a composition or a pharmaceutical composition containing the peptide of the present invention as an active ingredient activates β-catenin in hair follicle dermal papilla cells (HFDPCs) to promote nuclear translocation thereof.

In one embodiment, a composition or a pharmaceutical composition containing the peptide of the present invention as an active ingredient upregulates gene expression of one or more factors selected from the group consisting of LEF-1, c-Myc, and Cyclin D1, which are downstream factors of β-catenin, in hair follicle dermal papilla cells (HFDPCs).

In one embodiment, a composition or a pharmaceutical composition containing the peptide of the present invention as an active ingredient downregulates an expression level of DKK-1 (Dickkopf-1), which is a hair loss-inducing protein, in hair follicle dermal papilla cells (HFDPCs). The hair loss-inducing protein DKK-1 is a protein whose expression level is increased by dihydrotestosterone (DHT), and the peptide of the present invention may downregulate the expression level of DKK-1 that is increased by DHT.

In one embodiment, a composition or a pharmaceutical composition containing the peptide of the present invention as an active ingredient increases gene expression of one or more proteins selected from the group consisting of Ha3-II, Keratin 5, Keratin 14, and Keratin 19 in human hair outer root sheath cells (HHORSCs).

In one embodiment, a composition or a pharmaceutical composition containing the peptide of the present invention as an active ingredient increases expression of one or more transcription factors selected from the group consisting of MSX2, HOXC13, and FOXN1 in human hair germinal matrix cells (HHGMCs).

In one embodiment, a composition or a pharmaceutical composition containing the peptide of the present invention as an active ingredient downregulates expression of one or more stem cell markers selected from the group consisting of CD34, CD133, Keratin 15, and LHX2 in hair follicle stem cells (HFSCs).

In one embodiment, a composition or a pharmaceutical composition containing the peptide of the present invention as an active ingredient upregulates expression of one or more stem cell differentiation markers selected from the group consisting of α-SMA, Keratin 75, Vimentin, Versican, and Nexin-1 in hair follicle stem cells (HFSCs).

A composition or a pharmaceutical composition containing the peptide of the present invention as an active ingredient may include a therapeutically effective amount of the peptide of the present invention.

The term "therapeutically effective amount" refers to an amount sufficient for the peptide as an active ingredient to be contained in a composition to achieve its activity or efficacy, and for example, an amount sufficient to achieve efficacy in treating or preventing alopecia.

As used herein, the term "prevention" means reducing the risk of developing a disease or disorder, and refers to any action that suppresses or delays the onset of a disease by preventing the progression of the disease or one or more clinical signs thereof.

As used herein, the term "treatment" means alleviating a disease or disorder, and includes any action that improves or beneficially changes symptoms of the disease by arresting or reducing progression of the disease or one or more clinical signs thereof.

In the present invention, prevention or treatment of hair loss may include eliminating a cause of hair loss or inhibiting progression of hair loss, and may include inhibiting hair shedding or promoting hair formation to promote hair growth.

The composition or the pharmaceutical composition of the present invention may contain a pharmaceutically acceptable carrier.

The pharmaceutically acceptable carrier may be commonly used in formulation, and includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil.

The composition or the pharmaceutical composition of the present invention may further contain, in addition to the above ingredients, a lubricant, a wetting agent, a sweetening agent, a flavoring agent, an emulsifying agent, a suspending agent, a preservative, and the like, but is not limited thereto.

Suitable pharmaceutically acceptable carriers and formulations are described in detail in Remington: The Science and Practice of Pharmacy (19th ed., 1995, Williams & Wilkins).

The composition or the pharmaceutical composition of the present invention may be administered via any route suitable for treating alopecia, and may be administered, for example, orally or parenterally, and in the case of parenteral administration, by intravenous injection, subcutaneous injection, intramuscular injection, intraperitoneal injection, topical administration, transdermal administration, or the like. Since the composition or the pharmaceutical composition of the present invention has activity for preventing or treating alopecia, the composition or the pharmaceutical composition may be administered by topical application, such as application to the scalp.

The dosage of the composition or the pharmaceutical composition may be 0.0001 µg to 100 mg per day, 0.001 µg to 100 mg per day, 0.01 µg to 100 mg per day, 0.1 µg to 100 mg per day, or 1.0 µg to 1,000 mg per day, but is not limited thereto, and may be variously prescribed depending on factors such as formulation method, mode of administration, age, body weight, sex, pathological condition, food intake, administration time, route of administration, excretion rate, and responsiveness of a patient.

The composition or the pharmaceutical composition of the present invention may be formulated into a unit dosage form or prepared by being filled into a multi-dose container by using a pharmaceutically acceptable carrier and/or excipient according to a method that may be readily performed by those skilled in the art to which the present invention pertains. In this case, the formulation may be in the form of a solution, a suspension, or an emulsion in an oil or aqueous medium, or may be in the form of an extract, a powder, granules, tablets, or capsules, and may further include a dispersant or a stabilizer.

The composition or the pharmaceutical composition of the present invention may be a topical preparation. The topical preparation is a formulation in a form suitable for application to the exterior of the skin. When the composition or the pharmaceutical composition of the present invention is used as a topical preparation, it may be applied to the scalp, specifically to an area of the scalp where hair loss has occurred or to an area of the scalp where promotion of hair growth is desired. The topical preparation may be in the form of a cream, a gel, an ointment, a skin emulsion, a skin suspension, a transdermal delivery patch, a drug-containing bandage, a lotion, or a combination thereof. The topical preparation may be appropriately formulated, as necessary, with ingredients commonly used in topical preparations for cosmetics or pharmaceuticals, for example, aqueous ingredients, oily ingredients, powder ingredients, alcohols, moisturizers, thickening agents, ultraviolet absorbers, whitening agents, preservatives, antioxidants, surfactants, fragrances, colorants, various skin nutrients, or combinations thereof. The topical preparation may be appropriately formulated with metal chelating agents such as sodium edetate, trisodium edetate, sodium citrate, sodium polyphosphate, sodium metaphosphate, and gluconic acid, pharmaceutical agents such as caffeine, tannin, verapamil, licorice extract, glabridin, a hot-water extract of fruits of Kalin, various herbal medicines, tocopheryl acetate, glycyrrhizic acid, tranexamic acid, and derivatives or salts thereof, vitamin C, magnesium ascorbyl phosphate, ascorbyl glucoside, arbutin, kojic acid, sugars such as glucose, fructose, and trehalose, and the like, but is not limited thereto.

In still another aspect of the present invention, the composition of the present invention may be a cosmetic composition for promoting hair growth or preventing or alleviating alopecia, the cosmetic composition containing a peptide comprising the amino acid sequence of SEQ ID NO: 1 as an active ingredient.

In still another aspect of the present invention, the composition of the present invention may be a cosmetic composition for use in promoting hair growth or preventing or alleviating alopecia, the cosmetic composition containing a peptide comprising the amino acid sequence of SEQ ID NO: 1 as an active ingredient.

As used herein, the term "alleviation" refers to any action that reduces or relieves symptoms of a disease or disorder.

In one embodiment, a cosmetic composition containing the peptide of the present invention as an active ingredient promotes proliferation of hair follicle dermal papilla cells (HFDPCs) .

In one embodiment, a cosmetic composition containing the peptide of the present invention as an active ingredient promotes proliferation of hair outer root sheath cells (HORSCs).

In one embodiment, a cosmetic composition containing the peptide of the present invention as an active ingredient promotes proliferation of hair follicle stem cells (HFSCs).

In one embodiment, a cosmetic composition containing the peptide of the present invention as an active ingredient activates Akt or ERK, which are proteins involved in signaling, in hair follicle dermal papilla cells (HFDPCs).

In one embodiment, a cosmetic composition containing the peptide of the present invention as an active ingredient upregulates expression of growth factors in hair follicle dermal papilla cells (HFDPCs). The growth factors may be one or more selected from the group consisting of basic fibroblast growth factor (bFGF), hepatocyte growth factor (HGF), keratinocyte growth factor (KGF), and vascular endothelial growth factor (VEGF).

In one embodiment, a cosmetic composition containing the peptide of the present invention as an active ingredient activates β-catenin in hair follicle dermal papilla cells (HFDPCs) to promote nuclear translocation thereof.

In one embodiment, a cosmetic composition containing the peptide of the present invention as an active ingredient upregulates gene expression of one or more factors selected from the group consisting of LEF-1, c-Myc, and Cyclin D1, which are downstream factors of β-catenin, in hair follicle dermal papilla cells (HFDPCs).

In one embodiment, a cosmetic composition containing the peptide of the present invention as an active ingredient downregulates an expression level of DKK-1 (Dickkopf-1), which is a hair loss-inducing protein, in hair follicle dermal papilla cells (HFDPCs). The hair loss-inducing protein DKK-1 is a protein whose expression level is increased by dihydrotestosterone (DHT), and the peptide of the present invention may downregulate the expression level of DKK-1 that is increased by DHT.

In one embodiment, a cosmetic composition containing the peptide of the present invention as an active ingredient increases gene expression of one or more proteins selected from the group consisting of Ha3-II, Keratin 5, Keratin 14, and Keratin 19 in human hair outer root sheath cells (HHORSCs).

In one embodiment, a cosmetic composition containing the peptide of the present invention as an active ingredient increases expression of one or more transcription factors selected from the group consisting of MSX2, HOXC13, and FOXN1 in human hair germinal matrix cells (HHGMCs).

In one embodiment, a cosmetic composition containing the peptide of the present invention as an active ingredient downregulates expression of one or more stem cell markers selected from the group consisting of CD34, CD133, Keratin 15, and LHX2 in hair follicle stem cells (HFSCs).

In one embodiment, a cosmetic composition containing the peptide of the present invention as an active ingredient upregulates expression of one or more stem cell differentiation markers selected from the group consisting of α-SMA, Keratin 75, Vimentin, Versican, and Nexin-1 in hair follicle stem cells (HFSCs).

The cosmetic composition may be prepared in any formulation commonly manufactured in the technical field to which the present invention pertains, and may be a topical preparation. For example, the cosmetic composition may be formulated in the form of a solution, a suspension, an emulsion, a paste, a gel, a cream, a lotion, a powder, a soap, a surfactant-containing cleansing composition, an oil, a powder foundation, an emulsion foundation, a wax foundation, or a spray, but is not limited thereto.

The cosmetic composition may be prepared in various forms such as a solution, a sol-gel, an emulsion, an oil, a wax, and an aerosol, including, but not limited to, a softening toner, a nourishing toner, a nourishing cream, a massage cream, an essence, an eye cream, a cleansing cream, a cleansing foam, a cleansing water, a pack, a spray, a powder, a hair tonic, a hair cream, a hair lotion, a hair shampoo, a hair rinse, a hair conditioner, a hair spray, a hair aerosol, a pomade, and a gel.

The cosmetic composition of the present invention may contain other additives, such as excipients and carriers, and may be formulated by appropriately incorporating, as needed, conventional ingredients commonly used in general cosmetic compositions.

When the cosmetic composition is in the form of a paste, a cream, or a gel, animal oils, vegetable oils, waxes, paraffin, starch, tragacanth, cellulose derivatives, polyethylene glycol, silicone, bentonite, silica, talc, zinc oxide, or the like may be used as carrier components.

When the cosmetic composition is in the form of a powder or a spray, lactose, talc, silica, aluminum hydroxide, calcium silicate, or polyamide powder may be used as carrier components, and particularly in the case of a spray, a propellant such as chlorofluorohydrocarbon, propane/butane, or dimethyl ether may be additionally included, but is not limited thereto.

When the cosmetic composition is in the form of a solution or an emulsion, a solvent, a solubilizer, or an emulsifier may be used as carrier components, and examples thereof include water, ethanol, isopropanol, ethyl carbonate, ethyl acetate, benzyl alcohol, benzyl benzoate, propylene glycol, 1,3-butylene glycol oil, glycerol aliphatic esters, polyethylene glycol, or fatty acid esters of sorbitan.

When the cosmetic composition is in the form of a suspension, liquid diluents such as water, ethanol, or propylene glycol, suspending agents such as ethoxylated isostearyl alcohol, polyoxyethyl sorbitol esters, and polyoxyethylene sorbitan esters, microcrystalline cellulose, aluminum metahydroxide, bentonite, agar, tragacanth, or the like may be used as carrier components.

When the cosmetic composition is a surfactant-containing cleansing composition, aliphatic alcohol sulfates, aliphatic alcohol ether sulfates, sulfosuccinic acid monoesters, isethionates, imidazolinium derivatives, methyl taurates, sarcosinates, fatty acid amide ether sulfates, alkylamidobetaines, aliphatic alcohols, fatty acid glycerides, fatty acid diethanolamides, vegetable oils, lanolin derivatives, ethoxylated glycerol fatty acid esters, or the like may be used as carrier components.

When the cosmetic composition is in the form of a hair shampoo, base ingredients for formulating a shampoo, such as a thickening agent, a surfactant, a viscosity control agent, a moisturizer, a pH control agent, a preservative, and essential oils, may be mixed with the peptide of the present invention. As the thickening agent, CDE may be used; as the surfactant, an anionic surfactant, LES, and an amphoteric surfactant, coco betaine, may be used; as the viscosity control agent, polyquaternium may be used; as the moisturizer, glycerin may be used; and as the pH control agent, citric acid or sodium hydroxide may be used. As the preservative, grapefruit extract or the like may be used, and in addition, essential oils such as cedarwood, peppermint, and rosemary, as well as silk amino acids, pentaol, or vitamin E, may be added.

The ingredients contained in the cosmetic composition may further include, in addition to the peptide of the present invention as an active ingredient and carrier components, ingredients commonly used in cosmetic compositions, for example, conventional auxiliaries such as antioxidants, stabilizers, solubilizers, vitamins, pigments, or fragrances, but are not limited thereto.

The peptide of the present invention may be contained in the above-described composition at a concentration of 0.01 µM to 1,000 uM. Specifically, the peptide of the present invention may be contained at a concentration of 0.01 µM to 1,000 µM; 0.05 µM to 800 µM, 0.05 µM to 700 µM, 0.05 µM to 600 µM, 0.05 µM to 500 µM, 0.05 µM to 300 µM, or 0.05 µM to 200 µM; 0.1 µM to 800 µM, 0.1 µM to 700 µM, 0.1 µM to 600 µM, 0.1 µM to 500 µM, 0.1 µM to 300 µM, or 0.1 µM to 200 µM; 1 µM to 800 µM, 1 µM to 700 µM, 1 µM to 600 µM, 1 µM to 500 µM, 1 µM to 300 µM, or 1 µM to 200 µM; or 5 µM to 800 µM, 5 µM to 700 µM, 5 µM to 600 µM, 5 µM to 500 µM, 5 µM to 300 µM, or 5 µM to 200 µM, but is not limited thereto.

Among the terms or elements mentioned in the section "2. Composition for Promoting Hair Growth or Preventing, Alleviating, or Treating Alopecia", those that are the same as the terms or elements mentioned in the section "1. Peptide and Activity Thereof" are understood to be as described in the section "1. Peptide and Activity Thereof".

### 3. Use of Peptide of the Present Invention

In still another aspect of the present invention, there is provided use of a peptide comprising the amino acid sequence of SEQ ID NO: 1 for promoting hair growth, or inhibiting, preventing, alleviating, or treating alopecia.

In still another aspect of the present invention, there is provided a method for promoting hair growth or inhibiting, preventing, alleviating, or treating alopecia, the method including applying, to a subject in need of promoting hair growth or inhibiting, preventing, alleviating, or treating alopecia, a peptide comprising the amino acid sequence of SEQ ID NO: 1, or a composition containing the peptide as an active ingredient.

In the method, the applying may include administration, injection, infusion, inoculation, or application (for example, application to the skin), but is not limited thereto. The skin includes the scalp.

The subject refers to a subject in need of promoting hair growth or inhibiting, preventing, alleviating, or treating alopecia, and more specifically, may refer to a mammal such as a human or a non-human primate, a mouse, a rat, a dog, a cat, a horse, or a cow.

In still another aspect, there is provided use of a peptide comprising the amino acid sequence of SEQ ID NO: 1 for the manufacture of a medicament or cosmetics for promoting hair growth, or inhibiting, preventing, alleviating, or treating alopecia.

Among the terms or elements mentioned in "3. Use of Peptide of the Present Invention", those that are the same as those mentioned in "1. Peptide and Activity Thereof" or "2. Composition for Promoting Hair Growth or Preventing, Alleviating, or Treating Alopecia" are understood to be the same as those described above in "1. Peptide and Activity Thereof" or "2. Composition for Promoting Hair Growth or Preventing, Alleviating, or Treating Alopecia".

### ADVANTAGEOUS EFFECTS

The peptide of the present invention has activity of promoting hair growth, or preventing, inhibiting, or treating alopecia. The peptide of the present invention promotes proliferation of human follicle dermal papilla cells (HFDPCs), hair outer root sheath cells (HORSCs), and hair follicle stem cells (HFSCs), upregulates expression of growth factors such as bFGF, KGF, and VEGF in human follicle dermal papilla cells (HFDPCs), and downregulates an expression level of DKK-1 (Dickkopf-1), which is a hair loss-inducing protein. In addition, the peptide of the present invention has activity of increasing expression of keratin proteins in hair outer root sheath cells (HORSCs), increasing expression of transcription factors involved in cell activation in hair germinal matrix cells (HGMCs), and promoting differentiation of hair follicle stem cells (HFSCs).

However, the effects of the present invention are not limited to the effects mentioned above, and other effects that are not mentioned may be obviously understood by those skilled in the art from the following description.

### BRIEF DESCRIPTION OF THE DRAWINGS

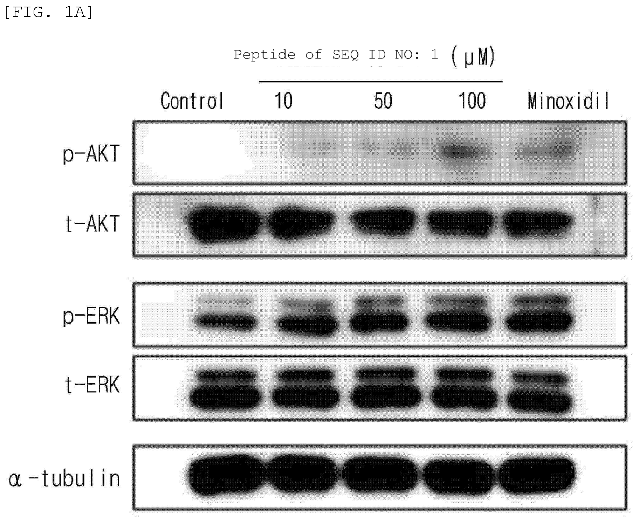

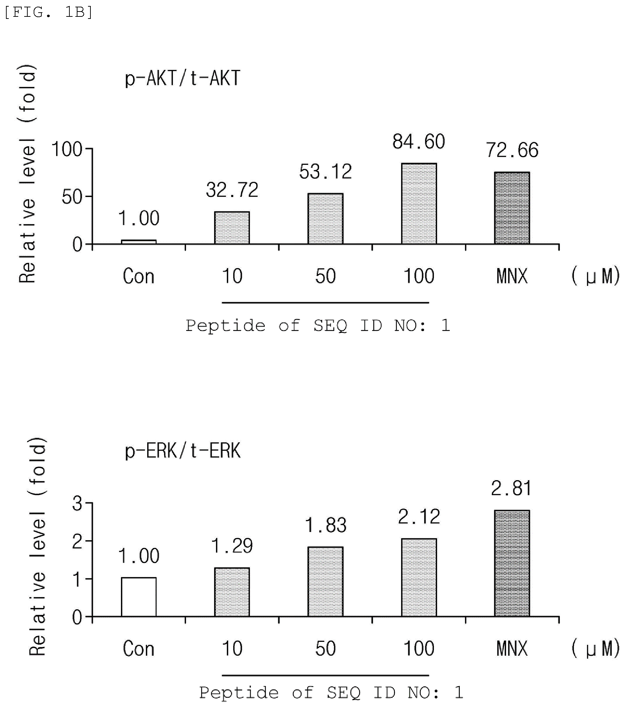

FIGS. 1A and 1B show experimental results demonstrating increased phosphorylation of Akt and extracellular-regulated kinase (ERK), which are signaling molecules related to cell proliferation, when HHFDPC cells are treated with the peptide of the present invention at 10 µM, 50 µM, and 100 µM. In FIGS. 1A and 1B, p-Akt and p-ERK indicate phosphorylated proteins of Akt and ERK, respectively, and t-Akt and t-ERK indicate total expressed proteins of Akt and ERK, respectively.

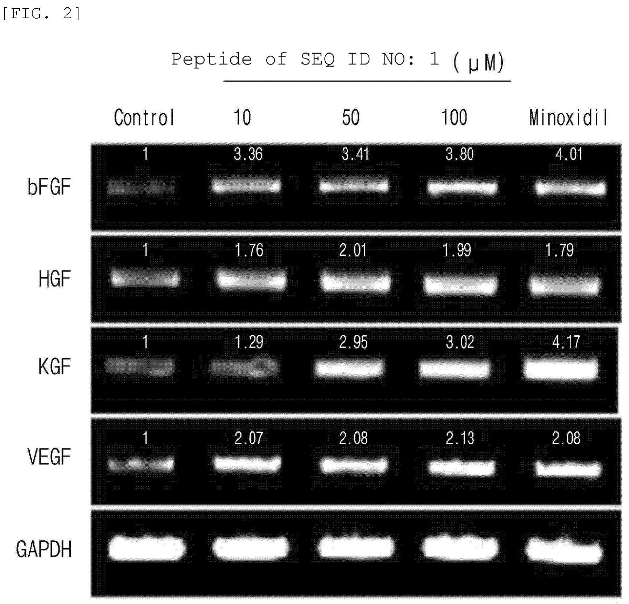

FIG. 2 shows experimental results demonstrating that, when HHFDPC cells are treated with the peptide of the present invention at 10 µM, 50 µM, and 100 µM, activity of HHFDPCs is increased, thereby increasing expression of growth factors such as bFGF, HGF, KGF, and VEGF. The numerical values shown above the bands in the drawing are relative values of band intensities of the respective genes, which are obtained by calculating band intensity values of the respective genes relative to a band intensity value of GAPDH and normalizing the control to 1.

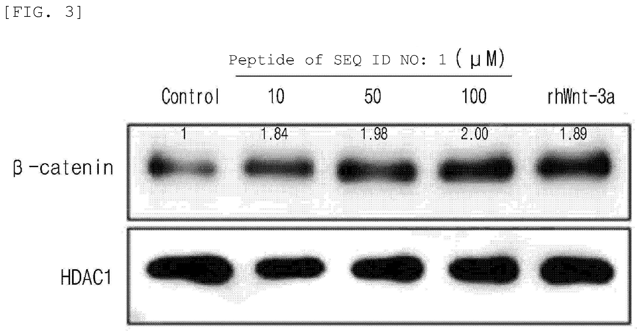

FIG. 3 shows experimental results demonstrating that, when HHFDPC cells are treated with the peptide of the present invention at 10 µM, 50 µM, and 100 µM, β-catenin is activated and nuclear translocation thereof is increased. The numerical values shown above the bands in the drawing are relative values of band intensities of β-catenin, which are obtained by calculating band intensity values of β-catenin relative to a band intensity value of HDAC1 and normalizing the control to 1.

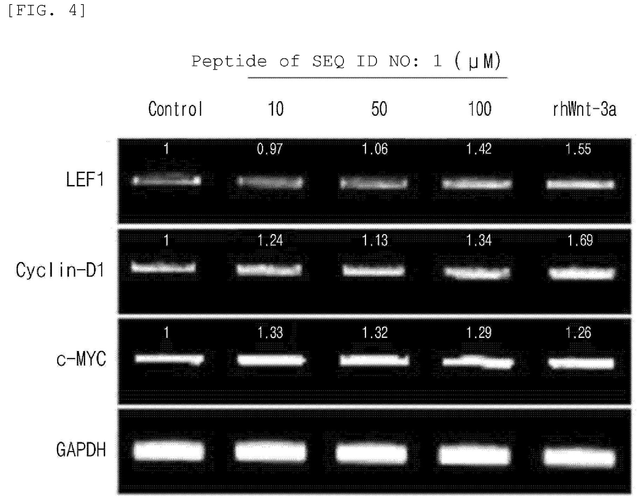

FIG. 4 shows experimental results demonstrating that, when HHFDPC cells are treated with the peptide of the present invention at 10 µM, 50 µM, and 100 µM, gene expression of LEF-1, Cyclin D1, and c-Myc, which are downstream factors of β-catenin, is increased. The numerical values shown above the bands in the drawing are relative values of band intensities of the respective genes, which are obtained by calculating band intensity values of the respective genes relative to a band intensity value of GAPDH and normalizing the control to 1.

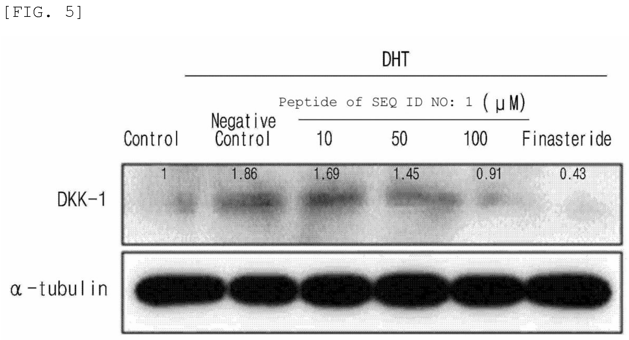

FIG. 5 shows experimental results demonstrating that, when HHFDPC cells are treated with the peptide of the present invention at 10 µM, 50 µM, and 100 µM, expression of DKK-1, which is a hair loss-inducing protein induced by DHT treatment, is decreased in a concentration-dependent manner. The numerical values shown above the bands in the drawing are relative values of band intensities of DKK-1, which are obtained by calculating band intensity values of DKK-1 relative to a band intensity value of α-tubulin and normalizing the control to 1.

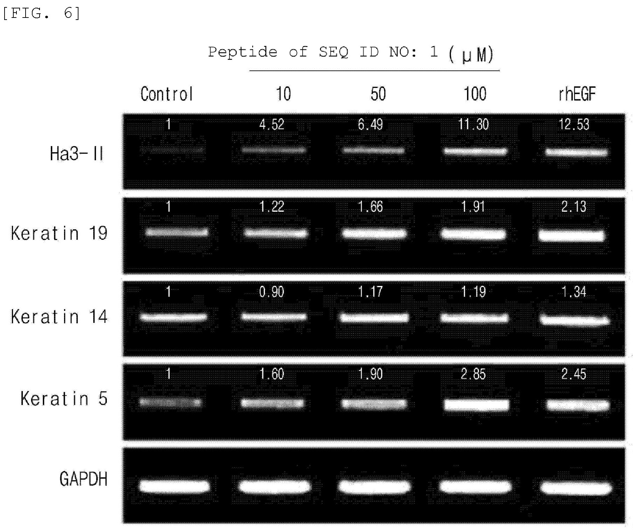

FIG. 6 shows experimental results demonstrating that, when HHORSC cells are treated with the peptide of the present invention at 10 µM, 50 µM, and 100 µM, cell activity is increased, thereby increasing gene expression of cytokeratin proteins Ha3-II, Keratin 19, Keratin 14, and Keratin 5. The numerical values shown above the bands in the drawing are relative values of band intensities of the respective genes, which are obtained by calculating band intensity values of the respective genes relative to a band intensity value of GAPDH and normalizing the control to 1.

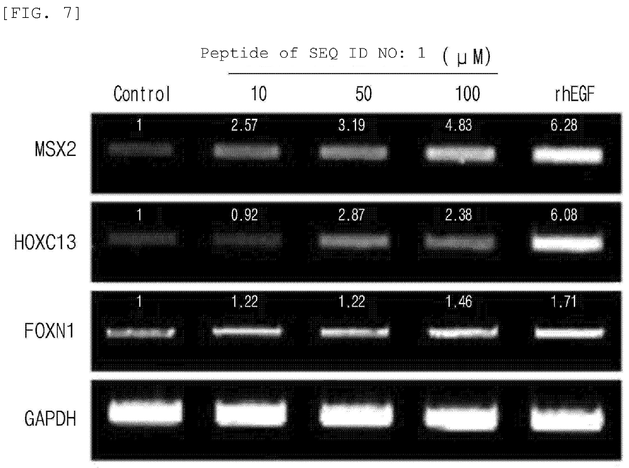

FIG. 7 shows experimental results demonstrating that, when HHGMC cells are treated with the peptide of the present invention at 10 µM, 50 µM, and 100 µM, gene expression of transcription factors MSX2, HOXC13, and FOXN1, which are involved in cell activity, is increased. The numerical values shown above the bands in the drawing are relative values of band intensities of the respective genes, which are obtained by calculating band intensity values of the respective genes relative to a band intensity value of GAPDH and normalizing the control to 1.

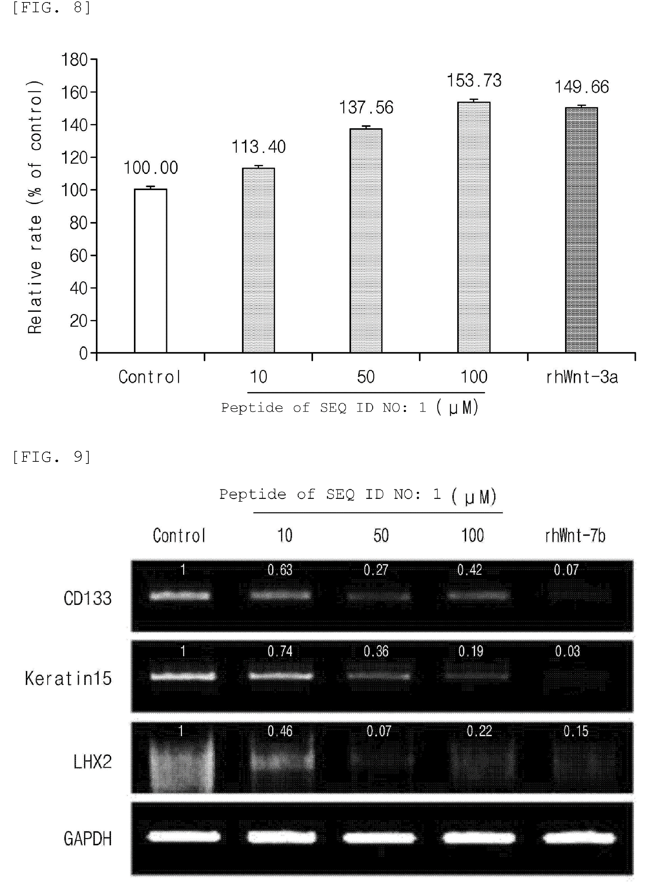

FIG. 8 shows experimental results demonstrating that, when HHFSC cells are treated with the peptide of the present invention at 10 µM, 50 µM, and 100 µM, proliferation of HHFSCs is promoted.
FIG. 9 shows experimental results demonstrating that, when HHFSC cells are treated with the peptide of the present invention at 10 µM, 50 µM, and 100 µM, gene expression of stem cell markers of HHFSCs, CD133, Keratin 15, and LHX2, is decreased. The numerical values shown above the bands in the drawing are relative values of band intensities of the respective genes, which are obtained by calculating band intensity values of the respective genes relative to a band intensity value of GAPDH and normalizing the control to 1.

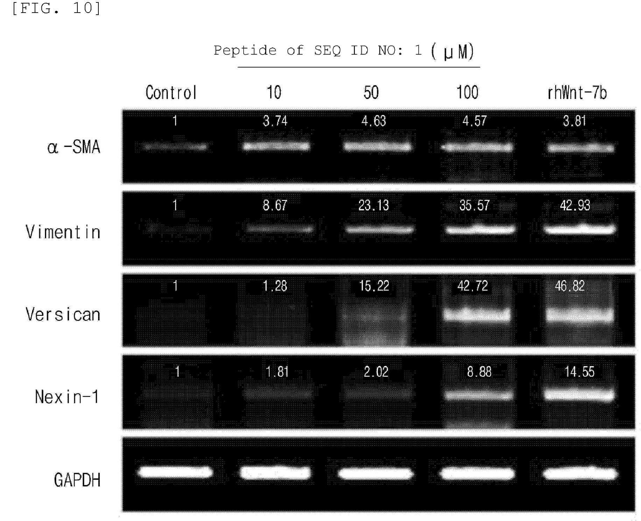

FIG. 10 shows experimental results demonstrating that, when HHFSC cells are treated with the peptide of the present invention at 10 µM, 50 µM, and 100 µM, gene expression of differentiation markers of HHFSCs, α-SMA, Vimentin, Versican, and Nexin-1, is increased. The numerical values shown above the bands in the drawing are relative values of band intensities of the respective genes, which are obtained by calculating band intensity values of the respective genes relative to a band intensity value of GAPDH and normalizing the control to 1.

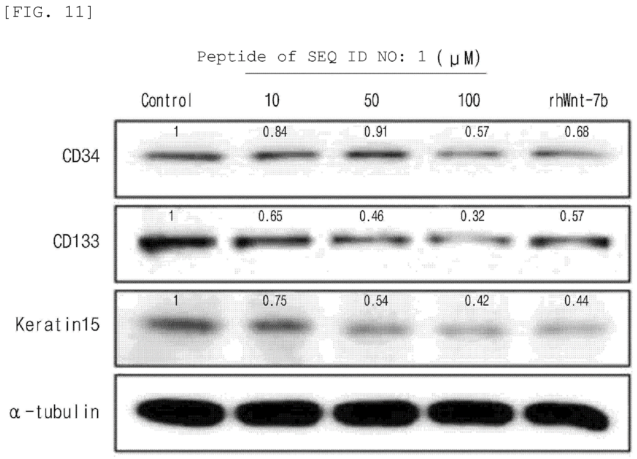

FIG. 11 shows experimental results demonstrating that, when HHFSC cells are treated with the peptide of the present invention at 10 µM, 50 µM, and 100 µM, expression of stem cell marker proteins of HHFSCs, CD34, CD133, and Keratin 15, is decreased. The numerical values shown above the bands in the drawing are relative values of band intensities of the respective markers, which are obtained by calculating band intensity values of the respective markers relative to a band intensity value of α-tubulin and normalizing the control to 1.

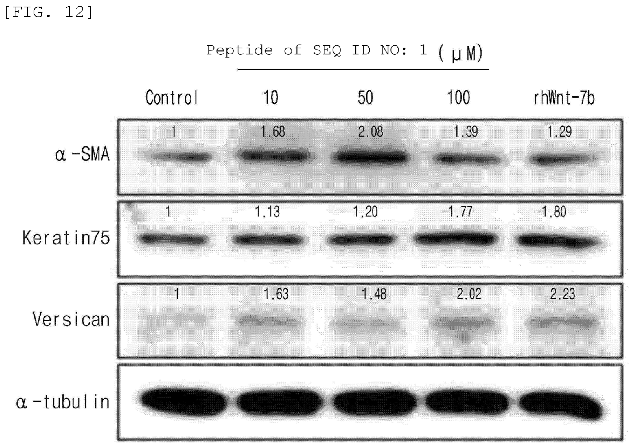

FIG. 12 shows experimental results demonstrating that, when HHFSC cells are treated with the peptide of the present invention at 10 µM, 50 µM, and 100 µM, expression of differentiation marker proteins of HHFSCs, α-SMA, Keratin 75, and Versican, is increased. The numerical values shown above the bands in the drawing are relative values of band intensities of the respective markers, which are obtained by calculating band intensity values of the respective markers relative to a band intensity value of α-tubulin and normalizing the control to 1.

### BEST MODE FOR CARRYING OUT THE INVENTION

Hereinafter, the present invention will be described in detail with reference to Examples. However, the following Examples are intended to specifically illustrate the present invention, and the present invention is not limited by the following Examples.

### [Examples]

### Preparation Example 1: Preparation of Peptide

Using an automated peptide synthesizer (Milligen 9050, Millipore, USA), peptides having the amino acid sequence of SEQ ID NO: 1 described in Table 1 were synthesized, and the synthesized peptides were isolated and purified by C18 reverse-phase high-performance liquid chromatography (HPLC) (Waters Associates, USA). As a column, an ACQUITY UPLC BEH300 C18 (2.1 mm × 100 mm, 1.7 um, Waters Co., USA) was used.

**[Table 1]**

| Amino acid sequence | SEQ ID NO: |
|---|---|
| DRFF | 1 |

In the following Experimental Examples, efficacy of the peptides prepared was evaluated.

### Experimental Example 1: Promotion of Proliferation of Human Hair Follicle Dermal Papilla Cells (HHFDPCs)

The effect of the peptide prepared in Preparation Example 1 on activation of proliferation-related signaling molecules in human hair follicle dermal papilla cells (HHFDPCs) was confirmed by a Western blot method.

HHFDPCs were seeded in 6-well cell culture plates at a density of 4 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with minoxidil (MNX) at 1 µM was used as a positive control, and an untreated cell culture was used as a control. The cell cultures were further cultured at 37°C for 24 hours. After washing the cultures with PBS, a lysis buffer was applied to lyse the cells, thereby obtaining cell lysates. BCA quantification was performed on the cell lysates, and protein samples having equal amounts were prepared. The prepared protein samples were subjected to electrophoresis using 10% SDS-PAGE. The proteins separated by SDS-PAGE were transferred to a PVDF membrane, and the membrane was blocked at room temperature for 30 minutes using 5% skim milk. Antibodies against phospho-AKT and phospho-ERK (Cell Signaling, USA) were diluted in 3% BSA at a ratio of 1:1000, and then reacted with the membrane at 4°C for 16 hours. The membrane was washed three times for 15 minutes each with 0.1% PBS-T (0.1% Tween-20 in PBS), and a secondary antibody diluted in 5% skim milk at a ratio of 1:2,000 was then reacted at room temperature for 1 hour. The membrane was washed three times for 15 minutes each with 0.1% PBS-T (0.1% Tween-20 in PBS) and treated with an ECL solution (GE Healthcare, USA), and expression levels were analyzed using an ImageQuant 800 (Cytiva, USA).

As a result of the experiment, as shown in FIGS. 1A and 1B, it was confirmed that, when HHFDPCs were treated with the peptide of Preparation Example 1 at 10 µM, 50 µM, and 100 µM, phosphorylation of Akt and extracellular signal-regulated kinase (ERK), which are signaling molecules related to cell proliferation, was increased.

### Experimental Example 2: Promotion of Gene Expression of Growth Factors in Human Hair Follicle Dermal Papilla Cells

The effect of the peptide prepared in Preparation Example 1 on expression levels of growth factors in human hair follicle dermal papilla cells (HHFDPCs) was confirmed by an RT-PCR method.

HHFDPCs were seeded in 6-well cell culture plates at a density of 3 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with minoxidil (MNX) at 1 µM was used as a positive control, and an untreated cell culture was used as a control. The cell cultures were further cultured at 37°C for 24 hours. After washing the cultures with PBS, 300 µL of easy blue (Intron, South Korea) was applied to isolate RNA. The isolated RNA was quantified using Nano drop, and cDNA synthesis was performed using a cDNA synthesis kit (Enzynomics, South Korea). Subsequently, a PCR reaction was performed using the primers shown in Table 2 and a PCR premix (Enzynomics, South Korea). The PCR reaction products were subjected to electrophoresis on a 1.5% agarose gel, and bands were detected using a Bio-Rad Gel Image System.

**[Table 2]**

| Gene | Primer | Sequence (5'→3') | SEQ ID NO: |
|---|---|---|---|
| bFGF | Forward | 5'-TGCTGGTGATGGGAGTTGTA-3' | 2 |
| | Reverse | 5'-CCTCCAAGTAGCAGCCAAAG-3' | 3 |
| HGF | Forward | 5'-CAATGCCTCTGGTTCCCCTT-3' | 4 |
| | Reverse | 5'-CCCCTCGAGGATTTCGACAG-3' | 5 |
| KGF | Forward | 5'-TCTGTCGAACACAGTGGTACCT-3' | 6 |
| | Reverse | 5'-GTGTGTCCATTTAGCTGATGCAT-3' | 7 |
| VEGF | Forward | 5'-ATGAACTTTCTGGGTCTTGGGT-3' | 8 |
| | Reverse | 5'-TGGCCTTGGTGAGGTTTGATCC-3' | 9 |
| GAPDH | Forward | 5'-GGAGCCAAAAGGGTCATCAT-3' | 10 |
| | Reverse | 5'-GTGATGGCATGGACTGTGGT-3' | 11 |

As a result of the experiment, as shown in FIG. 2, it was confirmed that, when HHFDPCs were treated with the peptide of Preparation Example 1 at 10 µM, 50 µM, and 100 µM, activity of HHFDPCs was increased, thereby promoting expression of growth factors such as bFGF, HGF, KGF, and VEGF.

### Experimental Example 3: Activation of β-Catenin in Human Hair Follicle Dermal Papilla Cells

The effect of the peptide prepared in Preparation Example 1 on activation of β-catenin in human hair follicle dermal papilla cells (HHFDPCs) was confirmed by a Western blot method.

HHFDPCs were seeded in 6-well cell culture plates at a density of 4 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with rhWnt-3a (recombinant human Wnt-3a protein, R&D Systems (USA, MN)) at 100 ng/mL was used as a positive control, and an untreated cell culture was used as a control. The cell cultures treated with the above substances were further cultured at 37°C for 24 hours. After washing the cell cultures with PBS, nuclear proteins were isolated using a nuclear protein extraction kit (Thermo Scientific, USA), and BCA quantification was performed to prepare protein samples having equal amounts. The prepared protein samples were subjected to electrophoresis using 10% SDS-PAGE. The proteins separated by SDS-PAGE were transferred to a PVDF membrane, and the membrane was blocked at room temperature for 30 minutes using 5% skim milk. Antibodies against β-catenin (Cell Signaling, USA) and HDAC1 (Santa Cruz, USA) were diluted in 3% BSA at a ratio of 1:1,000, and then reacted with the membrane at 4°C for 16 hours. The membrane was washed three times for 15 minutes each with 0.1% PBS-T (0.1% Tween-20 in PBS), and a secondary antibody diluted in 5% skim milk at a ratio of 1:2,000 was then reacted at room temperature for 1 hour. The membrane was washed three times for 15 minutes each with 0.1% PBS-T (0.1% Tween-20 in PBS) and treated with an ECL solution (GE Healthcare, USA), and expression levels were analyzed using an ImageQuant 800 (Cytiva, USA).

As a result of the experiment, as shown in FIG. 3, it was confirmed that, when HHFDPCs were treated with the peptide of Preparation Example 1 at 10 µM, 50 µM, and 100 µM, activation of the Wnt/β-catenin, which is known as an important signaling pathway related to hair growth, was observed, and nuclear translocation of β-catenin was markedly increased due to activation of β-catenin.

### Experimental Example 4: Promotion of Expression of β-Catenin Downstream Genes in Human Hair Follicle Dermal Papilla Cells

The effect of the peptide prepared in Preparation Example 1 on expression levels of lymphoid enhancer-binding factor 1 (LEF-1), Cyclin D1, and c-Myc, which are downstream genes of β-catenin, in human hair follicle dermal papilla cells (HHFDPCs) was confirmed by an RT-PCR method.

HHFDPCs were seeded in 6-well cell culture plates at a density of 4 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with rhWnt-3a (recombinant human Wnt-3a protein, R&D Systems (USA, MN)) at 100 ng/mL was used as a positive control, and an untreated cell culture was used as a control, and both were further cultured at 37°C for 24 hours. After washing the cultures with PBS, 300 µL of easy blue (Intron, South Korea) was applied to isolate RNA. The isolated RNA was quantified using Nano drop, and cDNA synthesis was performed using a cDNA synthesis kit (Enzynomics, South Korea). Subsequently, a PCR reaction was performed using the primers shown in Table 3 and a PCR premix (Enzynomics, South Korea). The PCR reaction products were subjected to electrophoresis on a 1.5% agarose gel, and bands were detected using a Bio-Rad Gel Image System.

**[Table 3]**

| Gene | Primer | Sequence | SEQ ID NO: |
|---|---|---|---|
| LEF-1 | Forward | 5'-CCAGCTATTGTAACACCTCA-3' | 12 |
| | Reverse | 5'-TTCAGATGTAGGCAGCTGTC-3' | 13 |
| cyclin-D1 | Forward | 5′- ACCTTCGTTGCCCTCTGTGCCACAGATG- 3′ | 14 |
| | Reverse | 5'-AGGCCCGGAGGCAGTCCGGGT-3' | 15 |
| c-Myc | Forward | 5′-ACCAGCAGCGACTCTGAGGAGGAAC- 3′ | 16 |
| | Reverse | 5'- TGACCCTCTTGGCAGCAGGATAGTCC-3'. | 17 |
| GAPDH | Forward | 5'-GGAGCCAAAAGGGTCATCAT-3' | 10 |
| | Reverse | 5'-GTGATGGCATGGACTGTGGT-3' | 11 |

As a result of the experiment, as shown in FIG. 4, it was confirmed that, when HHFDPCs were treated with the peptide of Preparation Example 1 at 10 µM, 50 µM, and 100 µM, β-catenin, which affects cell proliferation and differentiation, was activated, thereby increasing gene expression of downstream factors LEF-1, Cyclin D1, and c-Myc.

### Experimental Example 5: Inhibition of Expression of Hair Loss-Inducing Protein DKK-1 in Human Hair Follicle Dermal Papilla Cells

It is known that, in hair follicles, dihydrotestosterone (DHT) promotes expression of DKK-1 (Dickkopf-1), and DKK-1 induced and produced by DHT inhibits proliferation of keratinocytes in hair follicles and induces apoptosis thereof, thereby promoting hair loss (J of Invest Dermatolo, 2008, Feb., 128(2), pp. 262-269). The effect of the peptide prepared in Preparation Example 1 on the expression level of the hair loss-inducing protein DKK-1 in human hair follicle dermal papilla cells (HHFDPCs) was confirmed by a Western blot method.

HHFDPCs were seeded in 6-well cell culture plates at a density of 4 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively, and a cell culture treated with finasteride at 5 µM was used as a positive control. At this time, DHT, which is an inducing substance of the hair loss-inducing protein DKK-1, was simultaneously treated at a concentration of 100 nM. In addition, a cell culture treated with DHT alone was used as a negative control, and an untreated cell culture was used as a control. The cell cultures treated with the above substances were further cultured at 37°C for 24 hours. After washing the cultures with PBS, a lysis buffer was applied to lyse the cells, thereby obtaining cell lysates. BCA quantification was performed on the cell lysates, and protein samples having equal amounts were prepared. The prepared protein samples were subjected to electrophoresis using 10% SDS-PAGE. The proteins separated by SDS-PAGE were transferred to a PVDF membrane, and the membrane was blocked at room temperature for 30 minutes using 5% skim milk. An antibody against DKK-1 (Cell Signaling, USA) was diluted in 3% BSA at a ratio of 1:1,000, and then reacted with the membrane at 4°C for 16 hours. The membrane was washed three times for 15 minutes each with 0.1% PBS-T (0.1% Tween-20 in PBS), and a secondary antibody diluted in 5% skim milk at a ratio of 1:2,000 was then reacted at room temperature for 1 hour. The membrane was washed three times for 15 minutes each with 0.1% PBS-T (0.1% Tween-20 in PBS) and treated with an ECL solution (GE Healthcare, USA), and expression levels were detected using an ImageQuant 800 (Cytiva, USA).

As a result of the experiment, as shown in FIG. 5, it was confirmed that expression of DKK-1, which is a hair loss-inducing protein, was induced by treatment with DHT in HHFDPC cells, and that, when the peptide of Preparation Example 1 was treated at 10 µM, 50 µM, and 100 µM, expression of the hair loss-inducing protein DKK-1 induced by DHT treatment was decreased again in a concentration-dependent manner.

### Experimental Example 6: Activation of Human Hair Outer Root Sheath Cells (HHORSCs)

The effect of the peptide prepared in Preparation Example 1 on activation of human hair outer root sheath cells (HHORSCs) was confirmed by measuring gene expression of cytokeratin proteins.

HHORSCs were seeded in 6-well cell culture plates at a density of 4 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with rhEGF (recombinant human EGF protein) at 50 nM was used as a positive control, and an untreated cell culture was used as a control. The cell cultures were further cultured at 37°C for 24 hours. After washing the cultures with PBS, 300 µL of easy blue (Intron, South Korea) was applied to isolate RNA. The isolated RNA was quantified using Nano drop, and cDNA synthesis was performed using a cDNA synthesis kit (Enzynomics, South Korea). Subsequently, a PCR reaction was performed using the primers shown in Table 4 and a PCR premix (Enzynomics, South Korea). The PCR reaction products were subjected to electrophoresis on a 1.5% agarose gel, and bands were detected using a Bio-Rad Gel Image System.

**[Table 4]**

| Gene | Primer | Sequence | SEQ ID NO: |
|---|---|---|---|
| Ha3-II | Forward | 5'-GATCATCGAGCTGAGACGCA-3' | 18 |
| | Reverse | 5'-CTGGCCCCAGGGTATCTAGT-3' | 19 |
| Keratin 19 | Forward | 5'-CGCGGCGTATCCGTGTCCTC-3' | 20 |
| | Reverse | 5′-AGCCTGTTCCGTCTCAAACTTGGT- 3′ | 21 |
| Keratin 14 | Forward | 5'-CCAAATCCGCACCAAGGTCA-3' | 22 |
| | Reverse | 5'-GTATTGATTGCCAGGAGGGGG-3' | 23 |
| Keratin 5 | Forward | 5'-CCCTCAAGGATGCCAGGAAC-3' | 24 |
| | Reverse | 5'-CACTGCTACCTCCGGCAAG-3' | 25 |
| GAPDH | Forward | 5'-GGAGCCAAAAGGGTCATCAT-3' | 10 |
| | Reverse | 5'-GTGATGGCATGGACTGTGGT-3' | 11 |

As a result of the experiment, as shown in FIG. 6, it was confirmed that, when HHORSCs were treated with the peptide of Preparation Example 1 at 10 µM, 50 µM, and 100 µM, cell activity was increased, thereby increasing gene expression of cytokeratin proteins Ha3-II, Keratin 19, Keratin 14, and Keratin 5, which form the cellular cytoskeleton and contribute to hair formation.

### Experimental Example 7: Activation of Human Hair Germinal Matrix Cells (HHGMCs)

The effect of the peptide prepared in Preparation Example 1 on activation of human hair germinal matrix cells (HHGMCs) was confirmed by measuring gene expression of transcription factors Msh Homeobox 2 (MSX2), Homeobox C13 (HOXC13), and Forkhead Box N1 (FOXN1), which are related to cell activation.

HHGMCs were seeded in 6-well cell culture plates at a density of 4 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with rhEGF (recombinant human EGF protein) at 50 nM was used as a positive control, and an untreated cell culture was used as a control. The cell cultures were further cultured at 37°C for 24 hours. After washing the cultures with PBS, 300 µL of easy blue (Intron, South Korea) was applied to isolate RNA. The isolated RNA was quantified using Nano drop, and cDNA synthesis was performed using a cDNA synthesis kit (Enzynomics, South Korea). Subsequently, a PCR reaction was performed using the primers shown in Table 5 and a PCR premix (Enzynomics, South Korea). The PCR reaction products were subjected to electrophoresis on a 1.5% agarose gel, and bands were detected using a Bio-Rad Gel Image System.

**[Table 5]**

| Gene | Primer | Sequence | SEQ ID NO: |
|---|---|---|---|
| MSX2 | Forward | 5'-CGGTCAAGTCGGAAAATTCA-3' | 26 |
| | Reverse | 5'-GAGGAGCTGGGATGTGGTAA-3' | 27 |
| HOXC13 | Forward | 5'-GCCGTCTATACGGACATGCC-3' | 28 |
| | Reverse | 5'-GCAGTACACCTGACTGTCCC-3' | 29 |
| FOXN1 | Forward | 5'-CAGGACTGGGTGATGGTGTC-3' | 30 |
| | Reverse | 5'-TCTGAGGGTGAGAGCCTGAA-3' | 31 |
| GAPDH | Forward | 5'-GGAGCCAAAAGGGTCATCAT-3' | 10 |
| | Reverse | 5'-GTGATGGCATGGACTGTGGT-3' | 11 |

As a result of the experiment, as shown in FIG. 7, it was confirmed that, when HHGMCs were treated with the peptide of Preparation Example 1 at 10 µM, 50 µM, and 100 µM, gene expression of transcription factors MSX2, HOXC13, and FOXN1, which are involved in cell activation, was increased.

### Experimental Example 8: Promotion of Proliferation of Human Hair Follicle Stem Cells (HHFSCs)

The effect of the peptide prepared in Preparation Example 1 on proliferation of human hair follicle stem cells (HHFSCs) was examined.

HHFSCs were seeded in 96-well cell culture plates at a density of 2 × 10³ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with rhWnt-3a (recombinant human Wnt-3a protein) at 100 ng/ml was used as a positive control, and an untreated cell culture was used as a control. The cell cultures were further cultured at 37°C for 72 hours. Subsequently, 10 µL of a 5 mg/ml MTT solution was added thereto, and incubation was performed in a CO₂ incubator at 37°C for 4 hours. After removing the medium, 100 µL of DMSO was added, and shaking was performed for 10 minutes. Finally, absorbance at a wavelength of 540 nm was measured using a spectrophotometer (Molecular Devices (USA, CA) SpectraMax iD3).

As a result of the experiment, as shown in FIG. 8, it was confirmed that, when HHFSCs were treated with the peptide of Preparation Example 1 at 10 µM, 50 µM, and 100 µM, proliferation of HHFSCs was promoted in a concentration-dependent manner by up to 53.73% compared to a control.

### Experimental Example 9: Decrease in Expression of Stem Cell Marker-Related Genes in Human Hair Follicle Stem Cells

The effect of the peptide prepared in Preparation Example 1 on induction of differentiation of human hair follicle stem cells (HHFSCs) was confirmed by measuring gene expression of stem cell markers CD133, Keratin 15, and LIM homeobox 2 (LHX2).

HHFSCs were seeded in 6-well cell culture plates at a density of 1.5 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with rhWnt-7b (recombinant human Wnt-7b protein) at 50 ng/mL was used as a positive control, and an untreated cell culture was used as a control. The cell cultures were further cultured at 37°C for 24 hours. After washing the cultures with PBS, 300 µL of easy blue (Intron, South Korea) was applied to isolate RNA. The isolated RNA was quantified using Nano drop, and cDNA synthesis was performed using a cDNA synthesis kit (Enzynomics, South Korea). Subsequently, a PCR reaction was performed using the primers shown in Table 6 and a PCR premix (Enzynomics, South Korea). The PCR reaction products were subjected to electrophoresis on a 1.5% agarose gel, and bands were detected using a Bio-Rad Gel Image System.

**[Table 6]**

| Gene | Primer | Sequence | SEQ ID NO: |
|---|---|---|---|
| CD133 | Forward | 5'-GAGACCAAAGAGGCGTTGGA-3' | 32 |
| | Reverse | 5'-GGCTAGTTTTCACGCTGGTC-3' | 33 |
| Keratin 15 | Forward | 5'-CCAGCAAGACGGAGATCACA-3' | 34 |
| | Reverse | 5'-GAAGAGGCTTCCCTGATGGC-3' | 35 |
| Lhx2 | Forward | 5'-AAGCTGCAACGAAAACGACG-3' | 36 |
| | Reverse | 5'-GGTGGGGCTAGTCAAGTCTG-3' | 37 |
| GAPDH | Forward | 5'-GGAGCCAAAAGGGTCATCAT-3' | 10 |
| | Reverse | 5'-GTGATGGCATGGACTGTGGT-3' | 11 |

As a result of the experiment, as shown in FIG. 9, it was confirmed that, when HHFSCs were treated with the peptide of Preparation Example 1 at 10 µM, 50 µM, and 100 µM, gene expression of stem cell markers of HHFSCs, CD133, Keratin 15, and LHX2, was markedly decreased compared to a control. These results indicate that the peptide of Preparation Example 1 induces differentiation of HHFSCs, thereby decreasing expression of stem cell marker genes.

### Experimental Example 10: Increase in Expression of Stem Cell Differentiation Marker-Related Genes in Human Hair Follicle Stem Cells

The effect of the peptide prepared in Preparation Example 1 on induction of differentiation of human hair follicle stem cells (HHFSCs) was confirmed by measuring gene expression of differentiation markers α-SMA, Vimentin, Versican, and Nexin-1.

HHFSCs were seeded in 6-well cell culture plates at a density of 1.5 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with rhWnt-7b (recombinant human Wnt-7b protein) at 50 ng/mL was used as a positive control, and an untreated cell culture was used as a control. The cell cultures were further cultured at 37°C for 24 hours. After washing the cultures with PBS, 300 µL of easy blue (Intron, South Korea) was applied to isolate RNA. The isolated RNA was quantified using Nano drop, and cDNA synthesis was performed using a cDNA synthesis kit (Enzynomics, South Korea). Subsequently, a PCR reaction was performed using the primers shown in Table 7 and a PCR premix (Enzynomics, South Korea). The PCR reaction products were subjected to electrophoresis on a 1.5% agarose gel, and bands were detected using a Bio-Rad Gel Image System.

**[Table 7]**

| Gene | Primer | Sequence | SEQ ID NO: |
|---|---|---|---|
| α-SMA | Forward | 5'-CCTATCCCCGGGACTAAGAC-3' | 38 |
| | Reverse | 5'-ATGCTCTTCAGGGGCAACAC-3' | 39 |
| Vimentin | Forward | 5'-TGGACCAGCTAACCAACGAC-3' | 40 |
| | Reverse | 5'-GTCATTGTTCCGGTTGGCAG-3' | 41 |
| Versican | Forward | 5'-CCGTTCTCCCCAGGAAACTT-3' | 42 |
| | Reverse | 5'-TGCAGCGATCAGGTCGTTTA-3' | 43 |
| Nexin1 | Forward | 5'-CCCAGCCAACCTATGAGGAG-3' | 44 |
| | Reverse | 5'-CTTGAGGAGACCAGCACCAC-3' | 45 |
| GAPDH | Forward | 5'-GGAGCCAAAAGGGTCATCAT-3' | 10 |
| | Reverse | 5'-GTGATGGCATGGACTGTGGT-3' | 11 |

As a result of the experiment, as shown in FIG. 10, it was confirmed that, when HHFSCs were treated with the peptide of Preparation Example 1 at 10 µM, 50 µM, and 100 µM, gene expression of differentiation markers of HHFSCs, α-SMA, Vimentin, Versican, and Nexin-1, was markedly increased compared to a negative control. These results indicate that the peptide of Preparation Example 1 induces differentiation of HHFSCs, thereby increasing expression of differentiation marker genes.

### Experimental Example 11: Decrease in Expression of Stem Cell Markers in Human Hair Follicle Stem Cells

The effect of the peptide prepared in Preparation Example 1 on induction of differentiation of human hair follicle stem cells (HHFSCs) was confirmed by measuring protein expression of stem cell markers CD34, CD133, and Keratin 15.

HHFSCs were seeded in 6-well cell culture plates at a density of 3 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with rhWnt-7b (recombinant human Wnt-7b protein, R&D Systems (USA, MN)) at 50 ng/mL was used as a positive control, and an untreated cell culture was used as a control. The cell cultures treated with the above substances were further cultured at 37°C for 72 hours. After washing the cultures with PBS, a lysis buffer was applied to lyse the cells, thereby obtaining cell lysates. BCA quantification was performed on the cell lysates, and protein samples having equal amounts were prepared. The prepared protein samples were subjected to electrophoresis using 10% SDS-PAGE. The proteins separated by SDS-PAGE were transferred to a PVDF membrane, and the membrane was blocked at room temperature for 30 minutes using 5% skim milk. Antibodies against CD34, CD133, and Keratin 13 (Cell Signaling, USA) were diluted in 3% BSA at a ratio of 1:1,000, and then reacted with the membrane at 4°C for 16 hours. The membrane was washed three times for 15 minutes each with 0.1% PBS-T (0.1% Tween-20 in PBS), and a secondary antibody diluted in 5% skim milk at a ratio of 1:2,000 was then reacted at room temperature for 1 hour. The membrane was washed three times for 15 minutes each with 0.1% PBS-T (0.1% Tween-20 in PBS) and treated with an ECL solution (GE Healthcare, USA), and expression levels were analyzed using an ImageQuant 800 (Cytiva, USA).

As a result of the experiment, as shown in FIG. 11, it was confirmed that, when HHFSCs were treated with the peptide of Preparation Example 1 at 10 µM, 50 µM, and 100 µM, protein expression of stem cell markers CD34, CD133, and Keratin 15 was decreased. These results indicate that the peptide of Preparation Example 1 induces differentiation of HHFSCs, thereby decreasing expression of stem cell marker proteins.

### Experimental Example 12: Increase in Expression of Stem Cell Differentiation Markers in Human Hair Follicle Stem Cells

The effect of the peptide prepared in Preparation Example 1 on induction of differentiation of human hair follicle stem cells (HHFSCs) was confirmed by measuring protein expression of differentiation markers α-SMA, Keratin 75, and Versican.

HHFSCs were seeded in 6-well cell culture plates at a density of 3 × 10⁵ cells/well and cultured for 24 hours under conditions of mesenchymal stem cell complete media. Subsequently, the medium was replaced with a serum-free mesenchymal stem cell medium, and the cells were further cultured for 24 hours. The cell cultures were treated with the peptide prepared in Preparation Example 1 at 10 µM, 50 µM, and 100 µM, respectively. A cell culture treated with rhWnt-7b (recombinant human Wnt-7b protein, R&D Systems (USA, MN)) at 50 ng/mL was used as a positive control, and an untreated cell culture was used as a control. The cell cultures treated with the above substances were further cultured at 37°C for 72 hours. After washing the cultures with PBS, a lysis buffer was applied to lyse the cells, thereby obtaining cell lysates. BCA quantification was performed on the cell lysates, and protein samples having equal amounts were prepared. The prepared protein samples were subjected to electrophoresis using 10% SDS-PAGE. The proteins separated by SDS-PAGE were transferred to a PVDF membrane, and the membrane was blocked at room temperature for 30 minutes using 5% skim milk. Antibodies against α-SMA, Keratin 75, and Versican (Cell Signaling, USA) were diluted in 3% BSA at a ratio of 1:1,000, and then reacted with the membrane at 4°C for 16 hours. The membrane was washed three times for 15 minutes each with 0.1% PBS-T (0.1% Tween-20 in PBS), and a secondary antibody diluted in 5% skim milk at a ratio of 1:2,000 was then reacted at room temperature for 1 hour. The membrane was washed three times for 15 minutes each with 0.1% PBS-T (0.1% Tween-20 in PBS) and treated with an ECL solution (GE Healthcare, USA), and expression levels were analyzed using an ImageQuant 800 (Cytiva, USA).

As a result of the experiment, as shown in FIG. 12, it was confirmed that, when HHFSCs were treated with the peptide of Preparation Example 1 at 10 µM, 50 µM, and 100 µM, protein expression of stem cell differentiation markers α-SMA, Keratin 75, and Versican was markedly increased. These results indicate that the peptide of Preparation Example 1 induces differentiation of HHFSCs, thereby increasing expression of differentiation marker proteins.

### Preparation Example 2: Preparation of Pharmaceutical Composition

### 2-1. Preparation of Powder

Peptide of the present invention 2 g
Lactose 1 g

The above ingredients were mixed and filled into an airtight pouch to prepare a powder.

### 2-2 Preparation of Tablets

Peptide of the present invention 100 mg
Corn starch 100 mg
Lactose 100 mg
Magnesium stearate 2 mg

The above ingredients were mixed and then tableted according to a conventional tablet manufacturing method to prepare tablets.

### 2-3. Preparation of Capsules

Peptide of the present invention 100 mg
Corn starch 100 mg
Lactose 100 mg
Magnesium stearate 2 mg

The above ingredients were mixed and then filled into gelatin capsules according to a conventional capsule manufacturing method to prepare capsules.

### 2-4. Preparation of Pills

Peptide of the present invention 1 g
Lactose 1.5 g
Glycerin 1 g
Xylitol 0.5 g

The above ingredients were mixed and formed according to a conventional method so as to have a weight of 4 g per pill.

### 2-5. Preparation of Granules

Peptide of the present invention 150 mg
Soybean extract 50 mg
Glucose 200 mg
Starch 600 mg

The above ingredients were mixed, 100 mg of 30% ethanol was added thereto, and the mixture was dried at 60°C to form granules, which were then filled into a pouch.

### Preparation Example 3: Preparation of Cosmetic Composition

### 3-1. Preparation of Cream

Peptide of the present invention 4.6 parts by weight
Cetostearyl alcohol 2.8 parts by weight
Beeswax 2.6 parts by weight
Stearic acid 1.4 parts by weight
Lipophilic glyceryl monostearate 2 parts by weight
PEG-100 stearate 1 part by weight
Sorbitan sesquioleate 1.4 parts by weight
Jojoba oil 4 parts by weight
Squalane 3.8 parts by weight
Polysorbate 60 1.1 parts by weight
Macadamia oil 2 parts by weight
Tocopheryl acetate 0.2 parts by weight
Methyl polysiloxane 0.4 parts by weight
Ethyl paraben 0.1 parts by weight
Propyl paraben 0.1 parts by weight
Euxyl K-400 0.1 parts by weight
1,3-Butylene glycol 7 parts by weight
Methyl paraben 0.05 parts by weight
Glycerin 6 parts by weight
d-Panthenol 0.2 parts by weight
Triethanolamine 0.2 parts by weight
pt 41891 0.2 parts by weight
p-H2O 46.05 parts by weight

### 3-2. Preparation of Lotion

Peptide of the present invention 3.5 parts by weight
Cetostearyl alcohol 1.6 parts by weight
Stearic acid 1.4 parts by weight
Lipophilic glyceryl monostearate 1.8 parts by weight
PEG-100 stearate 2.6 parts by weight
Sorbitan sesquioleate 0.6 parts by weight
Squalane 4.8 parts by weight
Macadamia oil 2 parts by weight
Jojoba oil 2 parts by weight
Tocopheryl acetate 0.4 parts by weight
Methyl polysiloxane 0.2 parts by weight
Ethyl paraben 0.1 parts by weight
Propyl paraben 0.1 parts by weight
1,3-Butylene glycol 4 parts by weight
Methyl paraben 0.1 parts by weight
Xanthan gum 0.1 parts by weight
Glycerin 4 parts by weight
d-Panthenol 0.15 parts by weight
Allantoin 0.1 parts by weight
Carbomer (2% aq. Sol) 4 parts by weight
Triethanolamine 0.15 parts by weight
Ethanol 3 parts by weight
pt 41891 0.1 parts by weight
p-H2O 48.3 parts by weight

### 3-3. Preparation of Softening Toner

Peptide of the present invention 0.2 wt%
Ethanol 10.0 wt%
Polyoxyethylene sorbitan polylaurate 1.0 wt%
Methyl p-hydroxybenzoate 0.2 wt%
Glycerin 5.0 wt%
1,3-Butylene glycol 6.0 wt%
Fragrance q.s. (quantum satis)
Colorant q.s.
Purified water q.s.
Total 100

### 3-4. Preparation of Nourishing Toner

Peptide of the present invention 0.1 wt%
Petrolatum 2.0 wt%
Sorbitan sesquioleate 0.8 wt%
Polyoxyethylene oleyl ether 1.2 wt%
Methyl p-hydroxybenzoate q.s.
Propylene glycol 5.0 wt%
Ethanol 3.2 wt%
Carboxyvinyl polymer 18.0 wt%
Colorant q.s.
Fragrance q.s.
Purified water q.s.
Total 100

### 3-5. Preparation of Essence

Peptide of the present invention 5.0 wt%
Propylene glycol 10.0 wt%
Glycerin 10.0 wt%
Sodium hyaluronate aqueous solution (1%) 5.0 wt%
Ethanol 3.2 wt%
Polyoxyethylene hydrogenated castor oil 1.0 wt%
Methyl p-hydroxybenzoate 0.1 wt%
Fragrance q.s.
Purified water q.s.
Total 100

### 3-6. Preparation of Pack

Peptide of the present invention 0.5 wt%
Glycerin 5.0 wt%
Propylene glycol 4.0 wt%
Polyvinyl alcohol 15.0 wt%
Ethanol 8.0 wt%
Polyoxyethylene oleyl ether 1.0 wt%
Methyl p-hydroxybenzoate 0.2 wt%
Fragrance q.s.
Colorant q.s.
Purified water q.s.
Total 100

The above composition ratios are prepared by mixing suitable ingredients in preferred embodiments; however, the ingredients or mixing ratios may be arbitrarily modified depending on regional or ethnic preferences, such as target consumer groups, target countries, or intended uses.

Although representative embodiments of the present invention have been described above by way of example, the scope of the present invention is not limited to the specific embodiments described herein. Various modifications may be made by those skilled in the art within the scope of the claims of the present invention.

## Claims

1. A peptide comprising the amino acid sequence of SEQ ID NO: 1.

2. A composition for promoting hair growth or inhibiting, preventing, or alleviating alopecia, the composition comprising the peptide of claim 1 as an active ingredient.

3. A pharmaceutical composition for preventing or treating alopecia, the pharmaceutical composition comprising the peptide of claim 1 as an active ingredient.

4. The pharmaceutical composition of claim 3, wherein the peptide promotes proliferation of one or more cells selected from the group consisting of hair follicle dermal papilla cells (HFDPCs), hair outer root sheath cells (HORSCs), and hair follicle stem cells (HFSCs).

5. The pharmaceutical composition of claim 3, wherein the peptide, in hair follicle dermal papilla cells (HFDPCs),
(i) activates Akt or ERK signaling proteins;
(ii) upregulates gene expression of one or more factors selected from the group consisting of basic fibroblast growth factor (bFGF), hepatocyte growth factor (HGF), keratinocyte growth factor (KGF), and vascular endothelial growth factor (VEGF);
(iii) activates β-catenin;
(iv) upregulates gene expression of one or more β-catenin downstream factors selected from the group consisting of LEF-1, Cyclin D1, and c-Myc; or
(v) downregulates an expression level of hair loss-inducing protein DKK-1 (Dickkopf-1).

6. The pharmaceutical composition of claim 3, wherein the peptide, in hair outer root sheath cells (HORSCs), increases expression of one or more proteins selected from the group consisting of Ha3-II, Keratin 19, Keratin 14, and Keratin 5.

7. The pharmaceutical composition of claim 3, wherein the peptide, in hair germinal matrix cells (HGMCs), increases expression of one or more transcription factors selected from the group consisting of MSX2, HOXC13, and FOXN1.

8. The pharmaceutical composition of claim 3, wherein the peptide, in hair follicle stem cells (HFSCs),
(i) downregulates expression of one or more stem cell markers selected from the group consisting of CD34, CD133, Keratin 15, and LHX2; or
(ii) upregulates expression of one or more stem cell differentiation markers selected from the group consisting of α-SMA, Keratin 75, Vimentin, Versican, and Nexin-1.

9. The pharmaceutical composition of claim 3, wherein the pharmaceutical composition is a topical preparation.

10. A cosmetic composition for promoting hair growth or preventing or alleviating alopecia, the cosmetic composition comprising the peptide of claim 1 as an active ingredient.

11. The cosmetic composition of claim 10, wherein the peptide promotes proliferation of one or more cells selected from the group consisting of hair follicle dermal papilla cells (HFDPCs), hair outer root sheath cells (HORSCs), and hair follicle stem cells (HFSCs).

12. The cosmetic composition of claim 10, wherein the peptide, in hair follicle dermal papilla cells (HFDPCs),
(i) activates Akt or ERK signaling proteins;
(ii) upregulates gene expression of one or more factors selected from the group consisting of basic fibroblast growth factor (bFGF), keratinocyte growth factor (KGF), and vascular endothelial growth factor (VEGF);
(iii) activates β-catenin;
(iv) upregulates gene expression of one or more β-catenin downstream factors selected from the group consisting of LEF-1, c-Myc, and Cyclin D1; or
(v) downregulates an expression level of hair loss-inducing protein DKK-1 (Dickkopf-1).

13. The cosmetic composition of claim 10, wherein the peptide, in hair outer root sheath cells (HORSCs), increases expression of one or more proteins selected from the group consisting of Ha3-II, Keratin 5, Keratin 14, and Keratin 19.

14. The cosmetic composition of claim 10, wherein the peptide, in hair germinal matrix cells (HGMCs), increases expression of one or more transcription factors selected from the group consisting of MSX2, HOXC13, and FOXN1.

15. The cosmetic composition of claim 10, wherein the peptide, in hair follicle stem cells (HFSCs),
(i) downregulates expression of one or more stem cell markers selected from the group consisting of CD34, CD133, Keratin 15, and LHX2; or
(ii) upregulates gene expression of one or more stem cell differentiation markers selected from the group consisting of α-SMA, Keratin 75, Vimentin, Versican, and Nexin-1.

16. The cosmetic composition of claim 10, wherein the cosmetic composition is a topical preparation.

17. The cosmetic composition of claim 10, wherein the cosmetic composition has a formulation selected from the group consisting of a solution, a suspension, an emulsion, a paste, a gel, a cream, a lotion, a powder, a soap, a surfactant-containing cleansing composition, an oil, a powder foundation, an emulsion foundation, a wax foundation, and a spray.

18. The cosmetic composition of claim 10, wherein the cosmetic composition has a formulation selected from the group consisting of a hair tonic, a hair cream, a hair lotion, a hair shampoo, a hair rinse, a hair conditioner, a hair spray, a hair aerosol, a pomade, and a gel.

19. A method for promoting hair growth or inhibiting, preventing, alleviating, or treating alopecia, the method comprising applying a peptide comprising the amino acid sequence of SEQ ID NO: 1, or a composition containing the peptide as an active ingredient to a subject in need of promoting hair growth or inhibiting, preventing, alleviating, or treating alopecia.
